## Europäisches Patentamt

## European Patent Office

## Office européen des brevets

(19)

(11) Veröffentlichungsnummer: **0 255 604**

**A1**

(12) # EUROPÄISCHE PATENTANMELDUNG

(21) Anmeldenummer: **87109286.2**

(22) Anmeldetag: **27.06.87**

(51) Int. Cl.4: **A61K 7/08** , **A61K 7/06**

(30) Priorität: **05.07.86 DE 3622641**

(43) Veröffentlichungstag der Anmeldung:
**10.02.88 Patentblatt  88/06**

(84) Benannte Vertragsstaaten:
**AT BE CH DE ES FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Höffkes, Horst, Dr.**
**Carlo-Schmid-Strasse 113**
**D-4000 Düsseldorf-Hellerhof(DE)**
Erfinder: **Seidel, Kurt**
**Nosthoffenstrasse 59**
**D-4000 Düsseldorf 13(DE)**
Erfinder: **Giede, Karl**
**Schiehenweg 12**
**D-4010 Hilden(DE)**

(54) **Haarpflegemittel mit antistatischer Wirkung.**

(57) Durch die Verwendung quartärer Ammoniumverbindungen der Formel

$$R^1R^2R^3(\overset{+}{N})CH_3 \quad A^{(-)}$$

in der $R^1$, $R^2$ und $R^3$ Alkylgruppen mit 6 bis 12 C-Atomen sind, als antistatische Zusätze in haarkosmetischen Mitteln, läßt sich die elektrische Aufladbarkeit des Haars wirksam vermindern. Bevorzugt werden diese antistatischen Zusätze in Zusammensetzungen verwendet, die zusätzlich 5 bis 20 Gew.-% eines anionischen Tensids oder eines Gemisches aus anionischen und amphoteren, zwitterionischen oder Aminoxid-Tensiden enthalten.

EP 0 255 604 A1

### "Haarpflegemittel mit antistatischer Wirkung"

Gegenstand der Erfindung ist die Verwendung quartärer Ammoniumverbindungen, mit einer gegenüber bekannten kationischen Tensiden verstärkten antistatischen Wirkung auch in Gegenwart anionischer Tenside, in haarkosmetischen Mitteln.

Kationische Tenside vom Typ der quartären Ammoniumverbindungen werden in der Haarkosmetik als avivierende und konditionierende Wirkstoffe zur Verbesserung der Kämmbarkeit, der Fülle und des Griffes der Haare und zur Senkung der antistatischen Aufladbarkeit der Haare eingesetzt. Die Produkte werden meist in Haarnachbehandlungsmitteln verwendet, die nach der Haarwäsche dem Haar wieder günstige haarkosmetische Eigenschaften verleihen sollen. Sie eignen sich aber kaum als Zusatzmittel zu Haarwaschmitteln, um gleichzeitig mit der Wäsche einen gewissen konditionierenden Effekt zu erzielen, da die meisten bekannten quartären Ammoniumverbindungen mit anionischen Tensiden nicht verträglich sind, sondern haarkosmetisch unwirksame Niederschläge bilden. Quartäre Ammoniumverbindungen, die mit anionischen Tensiden verträglich sind, senken in der Regel die statische Aufladbarkeit des gewaschenen, trockenen Haares nur unzureichend.

Es bestand daher die Aufgabe, quartäre Ammoniumverbindungen zu finden, die starke avivierende und konditionierende Eigenschaften auf menschlichem Haar besitzen und die auch in Zusammensetzungen auf Basis schaumstarker anionischer Tenside, z. B. in Haarwaschmitteln (Shampoos) eingearbeitet werden können und dabei ihre gute, antistatische Wirksamkeit behalten.

Diese Anforderungen lassen sich erfindungsgemäß erfüllen durch die Verwendung quartärer Ammoniumverbindungen als antistatische Zusätze in haarkosmetischen Mitteln, wobei als quartäre Ammoniumverbindungen solche der Formel I

(I)  $R^1 R^2 R^{3(} \overset{+}{N} )CH_3$   $A^{(-)}$

in der $R^1 R^2$ und $R^3$ Alkylgruppen mit 6 bis 12 C-Atomen sind und $A^{(-)}$ ein Chlorid, Bromid oder ein Anion der Formel $C_n H_{2n+1} OSO_3^{(-)}$ ist, worin n = 1 oder 2 ist, neben üblichen Komponenten solcher Mittel verwendet werden. Verbindungen der Formel I sind literaturbekannt. Besonders bekannt sind die Produkte, in welchen $R^1$, $R^2$ und $R^3$ Alkylgruppen mit 8 bis 10 C-Atomen sind und die unter der Handelsbezeichnung Aliquat®336 (Henkel Corp.) oder Adogen®464 (Ashland Chem. Co.) erhältlich sind.

Die Produkte sind bei Raumtemperatur in Wasser nur begrenzt löslich, lassen sich aber in stabile wäßrige Dispersionen oder in wäßrig-alkoholische Lösung überführen. Erfindungsgemäße Haarpflegemittel können z. B. Haarwässer, Haarspülmittel, Haarkurpräparate, Haarfestiger, Fönwellotionen, Dauerwellfixierlotionen, Haarfärbemittel und Haarwaschmittel sein. Bevorzugt enthalten die erfindungsgemäßen Haarpflegemittel solche Verbindungen der Formel I, bei welcher $R^1$, $R^2$ und $R^3$ Alkylgruppen mit 8 bis 10 C-Atomen sind und A ein Chloridanion ist.

Die anwendungstechnischen Vorteile einer besonders guten antistatischen, d. h. die elektrische Aufladbarkeit des trockenen Haares vermindernden Wirkung werden am deutlichsten sichtbar in Haarpflegemitteln, die neben 0,01 bis 10 Gew.-% der quartären Ammoniumverbindung der Formel I 5 bis 20 Gew.-% eines anionischen Tensids oder eines Gemisches aus anionischen Tensiden einerseits und amphoteren, zwitterionischen oder Aminoxid-Ten siden andererseits enthalten. Solche Zubereitungen eignen sich zum Waschen der Haare. Aber auch Haarfärbemittel, z. B. Haarfärbeshampoos oder Oxidationshaarfärbecremes auf Basis einer anionischen Cremegrundlage und Oxidationsfarbstoffvorprodukten, die kurz vor der Anwendung mit einer wäßrigen Wasserstoffperoxid-Lösung zur Entwicklung der Oxidationsfarbstoffe vermischt und in die eigentliche Färbecreme überführt werden, lassen sich in ihren Anwendungseigenschaften erheblich verbessern, wenn man der Cremegrundlage eine quartäre Ammoniumverbindung der Formel I zusetzt.

Als anionische Tenside können alle für die Herstellung von Shampoos gebräuchlichen starkschäumenden synthetischen Aniontenside eingesetzt werden. Diese sind in erster Linie Alkylsulfate und Alkyl(poly)glycolethersulfate der Formel $R^4-O-(CH_2CH_2O)_x-SO_3^{(-)}M^{(+)}$ in der $R^4$ eine Alkylgruppe mit 10 bis 16 C-Atomen, x = 0 oder eine Zahl von 1 bis 12 und $M^{(+)}$ ein Alkali-, 1/2 $Mg^{(+)}$, Ammonium oder ein Mono-, Di-oder Trialkanolammonium-Kation mit 2 bis 4 C-Atomen in der Alkanolgruppe ist. Andere, für die erfindungsgemäßen Haarpflegemittel geeignete Aniontenside sind z. B. lineare Alkansulfonate und Alpha-Olefin-sulfonate mit jeweils 10 bis 18 C-Atomen, Sulfobernsteinsäuremono-und Dialkyl($C_8$-$C_{18}$)ester, Fettsäure($C_{12}$-$C_{18}$)alkanolamid-polyglycolethersulfate, Acylisethionate, Acyltauride und Acylsarkoside mit jeweils 12 bis 18 C-Atomen in der Acylgruppe.

In den erfindungsgemäßen Haarpflegemitteln können die anionischen Tenside teilweise ersetzt sein durch amphotere, zwitterionische oder Aminoxid-Tenside.

2

Amphotere Tenside sind z. B. N-Alkyl-ß-amino-propionsäuren, N-Alkyl-ß-iminodipropionsäuren, N-Hydroxyethyl-N-alkylamidopropyl-glycin, N-Alkyltaurin, N-Alkylsarkosin mit jeweils 8 bis 18 C-Atomen in der Alkylgruppe. Als zwitterionische Tenside eignen sich z. B. Betaintenside wie N-Alkyl-N,N-dimethyl-glycin, N-Acyl-aminopropyl-N,N-dimethylglycin mit jeweils 8 bis 18 C-Atomen in der Alkyl-bzw. Acylgruppe. Als Aminoxid-Tenside eignen sich z. B. Kokosamidopropyl-N,N-diemthylaminoxid oder N-Kokosalkyl-N,N-di(2-hydroxy)-ethyl-aminoxid.

Eine bevorzugte Ausführung der Erfindung sind konditionierende Haarshampoos, die 0,5 bis 5 Gew.-% der quartären Ammoniumverbindung der Formel I und 10 bis 20 Gew.-% eines Gemisches aus 60 bis 90 Gew.-% anionischen und 10 bis 40 Gew.-% amphoteren, zwitterionischen oder Aminoxid-Tensiden enthalten. In der Mischung aus anionischen und amphoteren, zwitterionischen oder Aminoxid-Tensiden ist die quartäre Ammoniumverbindung der Formel I besonders stabil dispergiert.

Neben den genannten Bestandteilen können die erfindungsgemäßen Haarpflegemittel alle für solche Formulierungen üblichen Hilfsmittel enthalten.

Shampoos können neben den genannten obligatorischen Komponenten noch weitere, z. B. nichtionogene Tenside, Verdickungsmittel, Schaumstabilisatoren, z. B. Fettsäurealkanolamide, Pflanzenextrakte, haarkosmetische Wirkstoffe wie z. B. Antischuppenwirkstoffe, Sebostatika und Proteinderivate enthalten.

Bei Haarspülmitteln und Haarkurpräparaten sind dies z. B. kosmetische Fett-und Ölkomponenten, Fettalkohole mit 16 bis 18 C-Atomen, Fettsäurepartialglyceride, z. B. Glycerinmono-und distearat, nichtionogene und kationische Tenside und haarkosmetische Wirkstoffe. Bei Haarwässern sind dies niedere Alkohole, haarkosmetische Wirkstoffe und ggf. Pflanzenextrakte. Bei Haarfestigern und Fönwellotionen können wasserlösliche Polymere mit festigender Wirkung enthalten sein. Haarfärbecremes enthalten schließlich die dafür üblichen Oxidationsfärbemittel-Vorprodukte in einem kosmetischen Träger, meist einer selbstemulgierfähigen Cremebasis, bestehend aus Fettalkoholen mit 12 bis 18 C-Atomen oder Fettsäurepartialglyceriden, ggf. Paraffinen und anionischen Tensiden, z. B. Seifen oder bevorzugt Fettalkoholsulfaten und/oder Fettalkoholpolyglycolethersulfaten auf Basis von Fettalkoholen mit 12 bis 18 C-Atomen und bis zu 12 Glycolethergruppen im Molekül oder Mischungen aus anionischen Tensiden und amphoteren, zwitterionischen oder Aminoxid-Tensiden.

Schließlich können alle erfindungsgemäßen Haarpflegemittel Farb-und Duftstoffe sowie Konservierungsmittel enthalten. Die nachfolgenden Beispiele sollen den Gegenstand der Erfindung näher erläutern.

## Beispiele

### 1. Messung der antistatischen Wirksamkeit

Ein Standardshampoo (1.1) und ein analog zusammengesetztes Shampoo mit 1 Gew.-% Aliquat®336 (1.2) wurden hergestellt und der folgenden Prüfung unterzogen:

### Prüfmethode

Standard-Haarsträhnen von 2 g Gewicht und 20 cm Länge der Firma Alkinco (Code 6634) wurden einmal blondiert und einmal kaltgewellt unter Verwendung handelsüblicher Präparate ohne quartäre Ammoniumverbindungen. Die so vorbehandelten Haarsträhnen wurden gewaschen, gespült und getrocknet und dann 90 Sekunden mit 0,5 ml einer 50%igen Lösung der Prüfshampoos in Leitungswasser bei 30 °C shamponiert.

Dann wurden die Haare mit Leitungswasser der gleichen Temperatur 30 Sekunden ausgespült.

Nach dem Trocknen (30 Minuten bei 60 °C) und Konditionieren der Haare bei 20 °C und 15 % rel. Luftfeuchtigkeit wurden die Haare (bei 15 % rel. Luftfeuchtigkeit) nach der Methode von A. C. Lunn und R. E. Evans (J. Soc. Cosmet. Chem. 28, 549 - 569 (Sept. 1977)) auf elektrostatische Aufladbarkeit untersucht. Dabei wurden die Haare 30 mal mit einem Hartgummikamm unter definierten Bedingungen durchgekämmt. Bei der mit dem Standard-Shampoo (1.1) behandelten Probe trat eine erhebliche statische Aufladung ein, die sich in einer starken Spreizung der Haarspitzen bemerkbar machte. Die mit dem Elektrometer gemessene Ladung für das Standardshampoo (1.1) ohne Kationtensid wurde = 100 % gesetzt. Die mit dem erfindungsgemäßen Shampoo (1.2) behandelte Haarsträhne zeigte unter den gleichen Bedingungen nur 7,2 % der statischen Ladung.

| Testshampoo Nr. | 1.1 | 1.2 |
|---|---|---|
|  | Gew.-% | Gew.-% |
| Fettalkohol$(C_{12}-C_{14})$ + 2 EO-sulfat, Na-Salz (28%ig) | 40 | 40 |
| N-Kokosacylaminopropyl-N,N-di-methylglycin (30%ig) | 10 | 10 |
| Aliquat$^{(R)}$336 | – | 1 |
| Wasser, entionisiert | 50 | 49 |
| statische Aufladbarkeit | 100 % | 7,2 % |

## 2. Beispiele für erfindungsgemäße Haarpflegemittel

### 2.1 Haarnachspülmittel
Cetyl-/Stearylalkohol 1,5 Gew.-%
Glycerinmono/di-palmitat/stearat 1,0 Gew.-%
Aliquat®336 2,0 Gew.-%
Cetyltrimethylammoniumchlorid 0,5 Gew.-%
Wasser ad 100 Gew.-%

### 2.2 Fönwellotion
Ethanol 30 Gew.-%
Luviskol®K30 0,5 Gew.-%
Gafquat®755 1,0 Gew.-%
Aliquat®336 0,1 Gew.-%
Wasser ad 100 Gew.-%

### 2.3 Haarfestiger
Isopropanol 40 Gew.-%
Luviset® CA66 2 Gew.-%
Triethanolamin 0,13 Gew.-%
Aliquat®336 0,05 Gew.-%
Wasser ad 100 Gew.-%

### 2.4 Haarfärbecreme (dunkelbraun)
Kokosfettalkohol $C_{12}-C_{18}$ 8,0 Gew.-%
Fettalkohol$(C_{12}-C_{14})$ + 2 EO-sulfat -Na-salz (28%ige Lösung) 20,0 Gew.-%
Aliquat®336 1,5 Gew.-%
$Na_2SO_3$ 1,0 Gew.-%
$(NH_4)_2SO_4$ 1,0 Gew.-%
p-Toluylendiamin 1,5 Gew.-%
2,4-Diaminoanisol 0,04 Gew.-%
p-Aminophenol 0,23 Gew.-%
Resorcin 0,4 Gew.-%
$NH_4OH$ (wäßrige Lösung) bis pH = 10
Wasser ad 100 Gew.-%

2.5 DauerwellfixierungEmulgade® F spezial     5 Gew.-%
Fettalkohol $C_{12}$-$C_{14}$ + 2 EO-sulfat Na-Salz (28%ige Lösung)     5 Gew.-%
Aliquat®336     1 Gew.-%
Wasser     69 Gew.-%

Natriumbromat     5 Gew.-%
Wasser     15 Gew.-%

In den Rezepturbeispielen wurden die folgenden Handelsbezeichnungen verwendet:
Luviskol®K30 (BASF):     Polyvinylpyrrolidon (K-Wert: 30 ± 3)
Gafquat®755 (GAF):     Vinylpyrrolidon-Dimethylaminoethylacrylat-Copolymer, mit Diethylsulfat quaterniert
Luviset®CA66 (BASF):     Vinylacetat-Crotonsäure-(9:1)-Copoylmerisat
Emulgade®F spezial (Henkel):     Gemisch aus Cetyl-/Stearyl-Alkohol und Rizinusöloxethylat
Aliquat®336 (Henkel Corp.):     Methyltri-n-alkyl($C_{8/10}$)ammoniumchlorid ($C_8$:$C_{10}$ = 2 : 1) (Mittleres Molekulargewicht 442)

## Ansprüche

1. Verwendung quartärer Ammoniumverbindungen als antistatische Zusätze in haarkosmetischen Mitteln, dadurch gekennzeichnet, daß als quartäre Ammoniumverbindungen solche der Formel I

(I)  $R^1 R^2 R^{3(}\overset{+}{N}{}^{)}CH_3$     $A^{(-)}$

in der $R^1 R^2$ und $R^3$ Alkylgruppen mit 6 bis 12 C-Atomen sind und $A^{(-)}$ ein Chlorid, Bromid oder ein Anion der Formel $C_nH_{2n+1}OSO_3^{(-)}$ ist, worin n = 1 oder 2 ist, verwendet werden.

2. Haarpflegemittel mit einem Gehalt an quartären Ammoniumverbindungen, dadurch gekennzeichnet, daß

0,01 bis 10 Gew.-% der quartären Ammoniumverbindung der Formel I gemäß Anspruch 1 und
5 bis 20 Gew.-% eines anionischen Tensids oder eines Gemisches aus anionischen und amphoteren, zwitterionischen oder Aminoxid-Tensiden
enthalten ist.

3. Haarshampoo mit einem Gehalt an quartären Ammoniumverbindungen, dadurch gekennzeichnet, daß

0,5 bis 5 Gew.-% der quartären Ammoniumverbindung der Formel I gemäß Anspruch 1 und
5 bis 20 Gew.-% eines Gemisches, bestehend aus 60 bis 90 Gew.-% anionischen und 10 bis 40 Gew.-% amphoteren, zwitterionischen oder Aminoxid-Tensiden
enthalten ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 144 688 (WELLA) <br> * Beispiel 7; Seite 2, Zeile 35 – Seite 3, Zeile 32; Ansprüche 1,5 * | 1 | A 61 K 7/08 <br> A 61 K 7/06 |
| Y | | 1-3 | |
| | --- | | |
| Y | FR-A-2 324 290 (KAO SOAP) <br> * Insgesamt * | 1-3 | |
| | --- | | |
| X,P | GB-A-2 177 109 (PROCTER & GAMBLE) <br> * Insgesamt * | 1-3 | |
| | ----- | | |

|  |
|---|
| **RECHERCHIERTE SACHGEBIETE (Int. Cl.4)** |
| A 61 K |

Der vorliegende Recherchenbericht wurde für alle Patentansprüche erstellt.

| Recherchenort | Abschlußdatum der Recherche | Prüfer |
|---|---|---|
| DEN HAAG | 16-11-1987 | FISCHER J.P. |